# EUROPEAN PATENT SPECIFICATION

(11) **EP 1 568 995 B1**
(45) Date of publication and mention of the grant of the patent: **28.10.2009**
(21) Application number: 03756935.7
(22) Date of filing: 14.01.2003
(51) Int. Cl.: G01N 33/543

(54) **NEW PROBE PLATE BASED ON ANTIGEN-ANTIBODY REACTION AND REAGENT KIT AND METHOD USING THE PROBE PLATE**
NEUE, AUF DER ANTIGEN-ANTIKÖRPER-REAKTION BERUHENDE SONDENPLATTE SOWIE REAGENZIENKIT UND VERFAHREN MIT VERWENDUNG DER SONDENPLATTE
NOUVELLE PLAQUE DE SONDES POUR REACTION ANTIGENE-ANTICORPS, TROUSSE DE REACTIF ET PROCEDE UTILISANT CETTE PLAQUE

(30) Priority: 06.06.2002 CN 02113834
(43) Date of publication of application: 31.08.2005
(73) Proprietor: Chengdu Kuachang Science & Technology Co., Ltd., Sichuan 610041 (CN)
(72) Inventor: ZOU, Fanglin, Chengdu, Sichuan 610000 (CN); CHEN, Chunsheng, Chengdu, Sichuan 610000 (CN); WANG, Jianxia, Sichuan 610000 (CN)
(74) Representative: Grünecker, Kinkeldey, Stockmair & Schwanhäusser Anwaltssozietät
(86) International application number: PCT/CN2003/000026
(87) International publication number: WO 2003/104808

(56) References cited:
- EP-A- 1 327 885
- EP-A- 1 348 961
- WO-A-02/077152
- WO-A-02/084249
- WO-A1-01/14425
- CN-A- 1 098 201
- CN-A- 1 184 935
- CN-A- 1 351 259
- CN-A- 1 373 365
- US-A- 5 763 158
- HUANG R-P: "Detection of multiple proteins in an antibody-based protein microarray system" JOURNAL OF IMMUNOLOGICAL METHODS, ELSEVIER SCIENCE PUBLISHERS B.V.,AMSTERDAM, NL, vol. 255, no. 1-2, 1 September 2001 (2001-09-01), pages 1-13, XP004274816 ISSN: 0022-1759
- WIESE R ET AL: "Simultaneous multianalyte ELISA performed on a microarray platform" CLINICAL CHEMISTRY, AMERICAN ASSOCIATION FOR CLINICAL CHEMISTRY. WINSTON, US, vol. 47, no. 8, August 2001 (2001-08), pages 1451-1457, XP002254091 ISSN: 0009-9147
- HAAB B B ET AL: "PROTEIN MICROARRAYS FOR HIGHLY PARALLEL DETECTION AND QUANTITATION OF SPECIFIC PROTENS AND ANTIBODIES IN COMPLEX SOLUTIONS" GENOME BIOLOGY (ONLINE), GB, vol. 2, no. 2, 2001, page COMPLETE, XP001147826 ISSN: 1465-6914
- ROWE C A ET AL: "AN ARRAY IMMUNOSENSOR FOR SIMULTANEOUS DETECTION OF CLINICAL ANALYTES" ANALYTICAL CHEMISTRY, AMERICAN CHEMICAL SOCIETY. COLUMBUS, US, vol. 71, no. 2, 15 January 1999 (1999-01-15), pages 433-439, XP000825701 ISSN: 0003-2700

## Description

### TECHNICAL FIELD

This invention pertains generally to probe-plate for qualitative and/or quantitative analysis for biological sample. In particular, the invention involves a probe-plate comprising a reactor used for qualitative and/or quantitative combination assay of different target molecules, e.g. target antibody and target antigen in one sample. In the reactor, different probes are immobilized to capture different target molecules. If marking is needed, different markers can be bound to the target molecules captured, thereby the reaction results can be expressed. This invention also involves a test kit comprising the probe-plate mentioned above, and a method using this probe-plate for biological sample analysis.

### BACKGROUND OF THE INVENTION

At present, antibody or antigen probe-plate, in which antibody or antigen is immobilized on a substrate, is widely used to detect the corresponding antibody or antigen, based on its specific reactivity. Three kind of representative kits for these using are: rapid test kit, routine test kit and biochip kit.

The present rapid test kit based on antibody-antigen reaction can provide a detecting result, identifiable to the unaided eyes. Its reactor usually comprises one strip of membranous matrix and the materials immobilized on the strip, such as antibody probe or antigen probe, control line and marker. In most of the detections, this kit acts upon an immune-affinity chromatography principle. And it is used for detecting target antigen or target antibody corresponding to the antibody probe or antigen probe immobilized. In some cases, the several reactors are combined to one probe-plate, each of the reactors comprising a test stripe detecting antigens or antibodies respectively. One example of this probe-plate with multi-reactors combined is that in a kit presented by Chinese patent application number 00226807.8(«A Biochip for Diagnosis and

Identification of Virus Hepatitis»). This combination of multi-rapid detection devices, or the probe-plate with multi-reactors combined, can be used for detecting the target hepatitis antigens and antibodies respectively. However, it is not different from the present rapid test kit. With it, one stripe (single device), namely one reactor in this rapid test kit detects only the target hepatitis antigens or antibodies, but not the target hepatitis antigens and antibodies.

The present routine test kits based the antibody-antigen reaction have the following characteristic: the probe is immobilized randomly in a reactor of its probe-plate. These kits include Enzyme-linked immunosorbent assay(ELISA) kit, biotin-avindin assay kit (e.g. biotin-avindin-substrate(BAS) solid-phase labeling kit), radio-immunoassay(RIA) kit, immune-fluorescence kit, chemiluminescence kit, electrochemiluminescence kit and etc. For example, the ELISA kit is composed of a probe-plate and a marking system. The ELISA probe-plate is usually a plastic or glass plate with several reactors, each of which comprises a microwell and an antibody or an antigen randomly immobilized on the inner surface of the microwell. The ELISA marking system contains an enzyme-labeled antigen or an enzyme-labeled antibody or an enzyme-labeled anti-antibody and a substrate. The kit is applied for detecting a target antibody or a target antigen in a sample, corresponding to the probe antibody or antigen immobilized.

With the routine test kits, the reaction among the reagents (probe molecules, target molecules, markers, etc.) in a reactor may be performed in different reaction patterns, such as indirect detection pattern, capturing method, competitive inhibition method, bi-antibody sandwich detection pattern and bi-antigen sandwich detection pattern etc. A probe-plate can be usually used for the different detection patterns.

For example, a probe-plate in ELISA kit can be used in different detection patterns according to the different marker in the marking system: if an specific antigen is immobilized on the antigen-coated plate, which will react with the target antibody in a sample, the bi-antigen sandwich detection pattern should be adopted in the case that the marker is an enzyme-labeled specific antigen, or the indirect detection pattern should be used if the marker is an enzyme-labeled anti-antibody; while for an antibody-coated plate, with a specific antibody immobilized which reacts with a target antigen in a sample, the bi-antibody sandwich detection pattern can be used if the marker is an enzyme-labeled specific antibody. No matter what kind of detection pattern is used, the target antibody or antigen can be detected qualitatively or quantitatively, after washing out unbound enzyme-labeled material, adding the substrates for coloring, and then detecting the OD value. So, the present ELISA kit and other routine test kits can only detect either antibodies or antigens by using one reactor. BioMerieux (France) has a kit that can simultaneously detect antigen and antibody (e.g. VIDAS HIV DUO); however, its reactions are performed respectively in two reactors, not in one reactor.

In this invention, the term "polypeptide chip" refers to "peptide arrays", "peptide microarrays", "peptide chips", "protein arrays" or "protein chips". At present, the polypeptide chips based on antibody-antigen reaction comprises one or more reactors, in which either multiple antigens or multiple antibodies are immobilized as the probes in a non-random form (e.g. in the address-targeted form). So, they are used for detecting either multiple antibodies or antigens in a sample, corresponding to the multiple antigens or multiple antibodies used as the probes. The biochip kit based on antibody-antigen reaction has extensive application, especially in the clinical immunology diagnosis. Although the research has been carried on for more than ten years, critical problems still exist for its large-scale application. The patterns of reaction among probes, targets and markers in a biochip reactor are almost the same as those in ELISA reactors, namely the indirect detection pattern, the capturing method, the competitive inhibition method, the bi-antibody sandwich detection pattern, the bi-antigen sandwich detection pattern and so on.

WO 02/077152 discloses a solid state substrate for testing against hepatitis surface antigen. EP 1348961 discloses a protein chip on which antibodies against tumor markers are immobilized, for use in detection of said tumor markers. WO 02/084249 discloses arrays bearing allergy-related antigens. WO 01/14425 discloses a protein chip bearing antigenic proteins originating from HBV, HIV or HCV. Huang (2001) J. Immunol. Methods 255, 1-13 discloses an antibody-based protein array bearing either antibodies or antigens (specifically cytokines). Wiese et al. (2001) Clin. Chem. 47, 1451-1457 discloses a protein array that allows simultaneous detection of multiple analytes (specifically PSA, PSA-ACT and IL-6) at multiple array addresses within a single well. Habb et al. (2001) Genome Biol. 2, 0004.1-0004.13 discloses protein microarrays for parallel detection and quantification of specific proteins and antibodies in complex solutions. Rowe et al. (1999) Anal. Chem. 71, 433-439 discloses an array immunosensor for simultaneous detection of clinical analytes (specifically staphylococcal enterotoxin B, F1 antigen from *Yersinia pestis* and D-dimer) in solutions. EP 1327885 discloses a diagnostic kit for the simultaneous detection of multiple infectious diseases via antibodies against disease-related antigens and the disease-related antigens themselves, including anti-HbsAg antibody, HCV antigen, syphilis antigen, HIV antigen. CN 1098201 A discloses an array for detection of HbsAg using an anti-HbsAg antibody.

In brief, the present test kits based on antibody-antigen consist of a probe-plate on which only antibody or antigen is immobilized in the reactor. So, a reactor can be only used for detecting either the target antigen or the target antibody.

Accordingly, the present invention provides a biochip kit, comprising:
- one biochip with at least one reactor, in which at least one probe antibody and one probe antigen are immobilized non-randomly in the reactor in the form of a probe micro-array, wherein said probe antibody includes a mouse antibody against anti-HIV p24 antigen and said probe antigen is a mouse multiple-epitope-mixing antigen comprising HIV gp36, gp41 and gp160; and
- marker, wherein said marker consists of ligands, corresponding to said probe antibody and probe antigen, and label reagent bound to the ligands.

In addition, the present invention provides a method for biological sample analysis employing the above biochip kit.

### Detailed description of the invention

Scientists have long recognized the following antigen-antibody reactivity: the specific reactivity between an antigen and its pair antibody; the species-specific reactivity based on different reactivities of an antibody with different anti-antibodies prepared from different species of animals; and the structure-specific reactivity based on different reactivities of a type or subtype of immunoglobulin with different anti-antibodies prepared by using different types or subtypes of immunoglobulin or their characteristic segments. However, this scientific knowledge has not been applied to the combination assay of different target molecules in one biochip reactor (e.g. antigen and antibody combination assay). To study the interaction among antigen/antibody/anti-antibody, we put this comparative knowledge into practice and invented said biochip kit. Some comparative results of this study are shown in Table 1.

**Table 1: The comparative experiment results on the cross-contamination among antigen/antibody/Anti-antibodies**

| reactor No. | probes | fluorescent marker | cross-contamination result* |
|---|---|---|---|
| 1 | rabbit antibody against HBsAg | Mouse mAbs against HBsAg | - |
| | Mouse mAbs against HBsAg | | - |
| 2 | Rabbit antibody against HBsAg | goat anti-Ab against human antibody | - |
| | Mouse mAbs against HBsAg | | - |
| 3 | anti-Ab against human IgM | purified human antibody against HBV | + |
| | anti-Ab against human IgG | | + |
| 4 | anti-Ab against human IgM | purified human IgG against HBV | - |
| | anti-Ab against human IgG | | + |
| 5 | anti-Ab against human IgM | purified human IgG against HBV | - |
| | HBsAg | | + |

| | | | |
|---|---|---|---|
| *:- means negative and + means positive | | | |

Said "probe-plate", such as multi-reactor biochip, is a chief component of kit, comprising one or more reactors,

Said "reactor", such as a chip-plate of a mono-reactor chip, a reaction well of a multi-reactor biochip, a coated well of an ELISA coated plate, and a test strip of a rapid test kit, refers to a boundary-defined continuous surface or container, where the probes are immobilized directly or indirectly and can react with target molecules of a subjected sample with identifiable reaction results.

The "antibody (Ab)" in said invention contains: natural antibody, specific antibody, polyclonal antibody, monoclonal antibody(mAb), genetic engineering antibody and antibody fragment. The "immunoglobulin (IG)" contains: different types of immunoglobulins (IgA, IgG, IgM, IgE, IgD), and different subtypes of immunoglobulins (IgG1, IgG2, IgG3, IgG4). The "antigen (Ag)" refers to an important substance in immunoassay, which can directly or indirectly stimulate immune response to produce antibodies and/or primed lymphocytes, and at the same time develop immunologic reaction by binding specifically to the immunity-responding products (i.e. antibody and/or primed lymphocyte).

The antigen in this invention contains: complete antigen, hapten and other substance with immune reactivity. Some examples of the antigen are the following: A. the component of plant, animal and microorganism with immunogenicity and immune reactivity (e.g. protein, peptides, lipid, polysaccharide, saccharide and other organic ingredients as well as combinations thereof); B. the metabolism products of plant, animal and microorganism; C. artificial antigen; D. synthetic antigen; E. genetic engineering recombinant antigen; F. medicine or ingredient of medicine; G. antibody used for preparing anti-antibody, or anti-antibody for preparing anti-anti-antibody.

In the present invention, the "substance to be marked" means a molecule to be made react with the marker. The substance to be marked can be a target antigen, a target antibody, or an intermediate connecting a target antigen or target antibody with the marker.

In the present invention, the "anti-antibody (anti-Ab)" means an antibody prepared from animal immunization by using another antibody or another anti-antibody as an antigen. The anti-antibody includes: antibody against an antibody, antibody against an anti-antibody, or antibody against an anti-anti-antibody, etc. The "probe of antibodies against immunoglobulins with different structural specificities" means antibodies prepared from animal immunization by using different structural immunoglobulins as antigen, which are used as the probes.

Some examples of the probes combination in this invention are showed as followings: a combination comprising antibody against HBsAg/HCV antigen/HTV₁₊₂ antigen/HTLV antigen/syphilis antigen (for the probes combination A); a combination comprising anti-antibody against IgM/Anti-antibody against IgG (for the probes combination B); a combination comprising goat Anti-antibody against human IgM/rabbit antibody against HBsAg/HCV antigen (for the probes combination C). More examples, some comparative, will be presented in the PART "examples".

In the reactor of said probe-plate, the probe combinations are immobilized either directly or indirectly. The immobilization of probes in the "indirect form" refers to an immobilization of probes on reactor through an intermediate, e.g. active molecule bound to a substrate, or activated particle to be bound to a substrate. The probes distribution in the reactor substrate can be either random or non-random. In a random distribution, the different probes immobilized cannot be identified geometrically. While the "non-random distribution" means a distribution by which the different probes immobilized can be identified geometrically. The non-random distribution contains address-traceable array or recognizable probe pattern.

Generally, the biochip comprises a reactor on which the probe antibody/antigen combination, or the probe anti-antibodies combination, is immobilized. An example for the former is a biochip comprising a reactor, in which M types of antigens (M ≧ 1) and N types of antibodies (N ≧ 1) are immobilized in the form of probe array, e.g. the blood transfusion scanning biochip in the example 1. An example for the latter is a biochip for identifying human immunoglobulin subtypes. This biochip comprises reactor with a probe combination of rabbit anti-antibodies against human IgG1/ IgG2/ IgG3/ IgG4 in the form of probe array. The rabbit anti-antibodies are prepared by using human IgG1/ IgG2/ IgG3/ IgG4 characteristic fragments to respectively immunize rabbits. This biochip can be used for a qualitative or quantitative deficit analysis of the human immunoglobulins subtypes of a patients, using fluorescence labeled goat anti-human IgG as the marker.

In the biochip of this invention, the probe can be immobilized either directly or indirectly on the reactor surface. The indirect immobilization of the probes can be performed as the following: firstly, different kinds of probes are immobilized on the activated particles separately, and then the activated particles bound to the probes are immobilized on the matrix of the biochip reactor. Whether the probes are immobilized directly or indirectly will not substantially affect the detection results of this probe-plate for different target molecules combinations described in the invention.

The biochip kit (or kit) of the subject invention comprises the probe-plate of the subject invention, with marking system. With this marking system, the reaction result in the probe-plate reactor is identifiable by detecting "mark" in the system either with unaided eyes or with photoelectric colorimeter, photoelectric imaging detector, fluorescence detector, radio-immunologic detector, micro-magnetic detector etc. A case in point is "the enzyme-labeled system" in the blood transfusion rapid detection (ELISA kit).

In this invention, the "test kit" or "kit" refers to an indispensable consumable in some quantitative and/or qualitative analysis, comprising one or more probe-plates with one or more reactors. The reactor, in which probes are immobilized and used for capturing sample target molecules, is a kernel of the kit.

The marking system in this invention is an important component of the kit. It refers to a mass of materials used to 'mark' the results of the reaction between the probes and the target molecules in a quantitative and/or qualitative assay. It includes ligands and label reagent, and optionally intermediates, label amplifying system and so on. The ligand in this invention refers to one or several molecules presenting affinity to the target molecules. An example of the marking system is that of an ELISA kit comprising an antigen-coated multi-well-plate in the indirect detection pattern, which contains the anti-antibodies labeled with enzyme and substrate.

The marking system in said kit comprises the ligands of the target molecules, the label reagent, and also in some detections the intermediates and the label amplifying system. Some examples of the label amplifying system are: the colloidal gold marking system with amplifying silver-salt, the marking system with biotin and/or avidin and so on.

The marker in said kit comprises the ligands of the target molecules and the label reagent bound thereto, such as the Anti-antibodies labeled with enzyme of an ELISA kit mentioned above. Another example of the marker is the fluorescence-labeled antibody. For example, the ligands of the markers of the ELISA kit in the EXAMPLE 12 are the specific antibody and the specific Anti-antibody, on which only the enzyme used as the label reagent. For example, in a fluorescence-labeled kit: the marker is a fluorescence-labeled antigen, a fluorescence-labeled antibody or a fluorescence-labeled anti-antibody; the ligand is the antibody, the antigen or the anti-antibody; the label reagent is the fluorescence reagent.

The marking system in said kit can use either different marking methods or different label reagents in the same marking method, e.g. two fluorescence reagents with different excitation and emission wavelength are used for detecting IgG and IgM respectively.

Each of the markers in said kit comprises the ligand with specific antibody-antigen reactivity or affinity to the antigen or/and antibody. In this invention, the affinity of the ligand to the antigen or/and antibody is defined as the specific reactivity of the ligand to the antigen or/and antibody. This affinity reactivity is based on affinity mechanisms rather than antigen-antibody reaction mechanism, such as the respective affinity of biotin, avidin, SPA, SPG, psoralen, digoxin with antigen and/or antibody.

In this invention, the "antigen-antibody specific reactivity" between an antigen and an antibody refers not only to the specific reactivity between an antigen and its pair antibody, but also to the species-specific reactivity and the structure-specific reactivity; the "species-specific reactivity" refers to the antigen-antibody reactivity based on different reactivities of an antibody with different anti-antibodies prepared from different species of animals; and the "structure-specific reactivity" refers to the antigen-antibody reactivity based on different reactivities of a type or subtype of immunoglobulin with different anti-antibodies prepared by using different types or subtypes of immunoglobulin or their characteristic segments. One example for the specific reactivity between an antibody and its pair antigen is that between human HCV antigen and pair human HCV antibody. One example for the species-specific reactivity is that a goat anti-antibody against human antibody reacts specifically with a human antibody against HBsAg but does not react significantly with a mouse mAb against HBsAg. One example for the structure-specific reactivity is that a human anti-HBV IgG has a significant reaction with an Anti-antibody against human IgG but not with an anti-antibody against human IgM.

The ligand of the marker in said kit is selected not only from the group of labeled specific antibody, labeled specific antigen and labeled anti-antibody, but also from the group of labeled anti-antibodies with species specificity and the group of labeled anti-antibodies with the structural specificity. The marker is to be one of the following marker combinations:
A. the combination of labeled specific antibody and labeled specific antigen;
B. the combination of labeled anti-antibodies with different structure-specificities ;
C. the combination of labeled anti-antibody with species-specificity and labeled specific antibody and/or labeled specific antigen;
D. the combination of labeled anti-antibody with structure-specificity and labeled specific antibody and/or labeled specific antigen;
E. any combination derived from the combinations mentioned-above.

Selection of the marker combinations (the sort and the number of the ligands labeled) depends on the sort and the number of target molecules to be detected and the detection pattern selected.

When test kit is used, the labeled specific antibody and antigen, the labeled species-specific anti-antibody and the labeled structure-specific anti-antibody in the marker combination can be added to the reactor respectively or mixed partially or wholely. An example for the combination A is the following combination: Rhodamine-labeled specific antigens (e.g.HCV, HIV, HTLV, syphilis specific antigens) and a Rhodamine-labeled specific antibody (e.g. an antibody against HBs). An example for the combination B is the following combination: a Rhodamine-labeled anti-antibody against IgM and a Rhodamine-labeled anti-antibody against IgG. An example for the combination C is the following combination: a Rhodamine-labeled goat anti-human Anti-antibody and a Rhodamine-labeled mouse mAbs against HBsAg. An example for the combination D is the following combination: a Rhodamine-labeled anti-antibody against IgM and a Rhodamine-labeled HBsAg.

The marking system in the subject polypeptide-chip kit adopts the following labeling methods or their combinations to mark the specific reactions: gold (silver) labeling method, enzyme labeling method, fluorescence labeling method, chemiluminescence labeling method, electrochemiluminescence labeling method, radioactive labeling method or magnetic labeling method. In said marking system, different label reagents can be used in one labeling method. For instance, different fluorescence reagents with different wavelength are bound to different ligands so as to form distinctive markers.

Some examples of the kit of this invention are showed as the following. These kits comprise a probe-plate, in which at least one probe combination mentioned above is immobilized randomly in at least one reactor are:
1)ELISA kit for antigen and antibody: it comprises a multi-well plate coated randomly with specific antigens and antibodies probes, enzyme-labeled specific antigens and antibodies or enzyme-labeled specific antigens and species-specific Anti-antibodies, substrate solution, buffer solution and other supplementary materials.
2)RIA kit: it consists of a multi-well plate coated randomly with specific antigens and antibodies probes, specific antigens and antibodies labeled with radioactive material, or specific antigen and species-specific Anti-antibodies labeled with radioactive material, buffer solutions and other supplementary materials.
3)RIA kit: it consists of a multi-well plate coated randomly with specific antigens and antibodies probes, specific antigens and antibodies labeled with radioactive material(radiation), or specific antigen and species-specific Anti-antibodies labeled with radioactive material, buffer solutions and other supplementary materials.
4)Electrochemiluminescence assay kit: it consists of a multi-well plate coated randomly with specific antigens and antibodies probes, specific antigens and antibodies labeled with Electrochemiluminescence or specific antigens and species-specific Anti-antibodies labeled with Electrochemiluminescence, buffer solutions and other supplementary materials
5)Chemiluminescence assay kit: it consists of a multi-well plate coated randomly with specific antigens and antibodies probes, specific antigens and antibodies labeled with Chemiluminescence or specific antigens and species-specific Anti-antibodies, buffer solutions and other supplementary materials.

These kits mentioned above are preferred in the test in which the result will be judged as unqualified if one detecting result is positive. Take blood transfusion test kit for example, when one of HBV, HCV, HIV, syphilis detecting result is positive, the blood donator and the bag of blood or plasma will be judged as unqualified.

In the conventional test kits of said invention, one of the following labeling materials or their combinations can be used to mark the specific reactions: gold (silver), enzyme, fluorescence, chemiluminescence, electrochemiluminescence, radiation or magnet. Employing the same labeling method, the ligand of the labeled materials can be paired with same label reagent or different label reagent. The label reagents include: radioactive material, enzyme, coloration substrate, biotin-avindin, SPA, SPG, psoralen, digoxin, fluorescent dye, colloidal gold (silver), rare earth compound colorant, chemiluminescent reagent, electrochemiluminescent reagent, bioluminescent reagent, magnetic particle and so on.

All the kits disclosed can be used to detect one of the following 6 different target molecule combinations:
A. the combination of antigen and antibody;
B. the combination of different types of immunoglobulins;
C. the combination of different subtypes of immunoglobulins;
D. the combination of antigen and different types of immunoglobulin and /or the mixture of different types of immunoglobulin;
E. the combination of antigen and different subtypes of immunoglobulin and /or the mixture of different types of immunoglobulin;
F. any derived combination thereof.

In this invented kit, the probes are immobilized on the solid-phase substrate of the reactor of the probe-plate. The solid-phase substrate in this invention includes various organic, inorganic, transparent or opaque, water-absorbable or water-unabsorbable, hard or soft materials, such as one or several kinds of following materials: glass, plastic, rubber, metal, cellulose membrane, slice, plate, etc. The shape of the probe-plate in this invention can be various, such as flat plate, pore plate and strips with various shape, etc. On the probe-plate in this invention, there could be one or several selective reactors. In each reactor, antigens and antibodies probes for detecting specific reaction are immobilized non-randomly. The specific antibodies or antigens immobilized on the probe-plate of this invention can be natural or synthetic materials including polypeptide, protein and all other substance with the properties of antibody or antigen. In the manufacture, various probes (specific antigens or specific antibodies) can be immobilized on the probe-plate simultaneously or successively.

The advantage of the kit and the method of this invention is: the same reactor can be used to test different target molecules such as antibody and antigen, different types of immunoglobulins, different subtypes of immunoglobulins etc.

Thereby the number of reactors and time needed by testing the combination are decreased, while the assay comparability is improved. The kit in this invention is economical, timesaving and efficient.

Followings are the detailed examples for specification of this invention. All of the activated substrates for the biochip, used in the examples 1-8 are available in domestic market or made and provided by SEDAC Corp. France.

### Comparative Example 1:

### A biochip screening kit for blood transfusion (1)

In this comparative example, the kit consists of screening biochip probe-plate for blood transfusion, markers, negative control, positive control, buffers for washing, etc.

The kit is to be used for detecting Hepatitis B surface antigen (HbsAg),

Hepatitis C virus (HCV) antibodies, HIV₁₊₂ antibodies and syphilis antibodies in human serum.

The probe combination used in the subject kit, designed in accordance with the blood screening items required by law, is that of one antibody and 3 antigens. The probe antibody selected is a rabbit antibody against human HBsAg. The probe antigens selected are respectively a multiple-epitope-fusion HCV antigen, a multiple-epitope-mixing HIV₁₊₂ antigen and a multiple-epitope-mixing syphilis antigen. The probes at its optimized concentration are respectively spotted on a substrate in the form of a 4X3 micro-array (each probe spotted in 3 spots), and then bovine albumin is used to inactivate residual surface of the substrate. The biochip probe-plate is then dried and ready for use.

The detection method used in the subject kit is a combination method of indirect detecting method and bi-antibody sandwich detection. The marker combination used in the subject kit is that of one labeled specific antibody and one labeled species-specific anti-antibody. The specific antibody, used as ligand in the marker combination, is a mouse mAb against HBsAg, which can specifically form a bi-antibody sandwich with the immobilized rabbit antibody against HBsAg and human HBsAg captured by the immobilized antibody. The anti-antibody, used as ligand in the anti-antibody, is a goat anti-antibody against human antibodies, which can bind with human HCV Ab, HIV₁₊₂ Ab and syphilis Ab, captured by their pair probe antigens (HCV Ag, HIV₁₊₂ Ag and syphilis Ag) immobilized respectively, but cannot bind with the probe antibody (the rabbit antibody against human HBsAg) and the labeled antibody (the mouse mAb against HBsAg). In this example, rhodamine is used as the label reagent and is bound to the ligands. The ligands are labeled with the rhodamine under optimized conditions, respectively. The markers prepared are ready for use in the form of a mixture or of two separated markers.

The negative control of this chip kit is a human serum negative in HBsAg, HCV antibody, HIV₁₊₂ antibody and syphilis antibody, selected by ELISA kits. The ELISA probe-plates used in the ELISA kits are micro-well plates coated respectively with rabbit antibody against HBsAg, multiple-epitope-fusion HCV antigen, multiple-epitope-mixing HIV₁₊₂ antigen or syphilis antigen. The positive control of this chip kit is a mixture of HBsAg positive sera, HCV antibody positive sera, HIV₁₊₂ antibody positive sera and syphilis antibody positive sera, selected by using the ELISA kits.

This biochip screening kit for blood transfusion can be used for detecting the possible presence of HBsAg, HCV Ab, HIV₁₊₂ Ab and syphilis Ab in a human serum sample. The human serum samples in this experiment are: 1.HbsAg positive human serum, 2.HCV antibody positive human serum, 3.HIV₁₊₂ antibody positive human serum, 4.Syphilis antibody positive human serum, 5.HbsAg positive and syphilis positive human serum, 6.Negative control and 7. Positive control. These human serum samples are previously detected using the ELISA kits.

The samples are detected as following: one of the human serum samples is diluted and subjected into one reactor of this chip. The antigen-antibody reaction in the reactor is performed at 37°C for one hour. Then, the unbound serum component is removed from the reactor and the reactor is washed with washing buffer. The marker combination (the mouse mAb against human HBsAg and goat anti-human anti-antibodies labeled with rhodamine) is then subjected into the reactor. The marking reaction is performed at 37°C for one hour. Then, the reactor is washed first with buffer solution to get rid of unbound markers, and then with anhydrous alcohol. Finally, the dried biochip probe-plate is scanned and analyzed with a chip scanner (GMS 418 ARRAY SCANNER). The results of the detections with this chip kit are showed in the Table 2.

**Table 2:**

| Detection results of the biochip screening kit for blood transfusion | | | | | | | | |
|---|---|---|---|---|---|---|---|---|
| Samples | results using 4 ELISA kits | | | | results using 1 biochip kit | | | |
| | HbsAg | HCV | HIV | syphilis | HbsAg | HCV | HIV | syphilis |
| 1 | +* | -* | - | - | + | - | - | - |
| 2 | + | + | - | - | + | - | - | - |
| 3 | - | - | + | - | - | - | + | - |
| 4 | - | - | - | + | - | - | - | + |
| 5 | + | - | - | + | + | - | - | + |
| Negative control | - | - | - | - | - | - | - | - |
| Positive control | + | + | + | + | + | + | + | + |
| Blank | - | - | - | - | - | - | - | - |

| | | | | | | | | |
|---|---|---|---|---|---|---|---|---|
| *: same as those in Table 1 | | | | | | | | |

### Comparative Example 2:

### A biochip screening kit for blood transfusion (2)

In this comparative example, the kit consists of the screening biochip probe-plate for blood transfusion, markers, negative control, positive control, washing solution, etc.

The biochip probe-plate in this biochip kit is prepared by the same preparation method as that in comparative example 1.

The detection method combination used in the subject kit is that of bi-antigen sandwich detection and bi-antibody sandwich detection. The marker combination used in the subject kit is that of three labeled specific antigens and one labeled specific antibody. The specific antibody, used as ligand in the marker combination, is a mouse mAb against HBsAg, which can specifically form a bi-antibody sandwich with the immobilized rabbit antibody against HBsAg and human HBsAg captured by the immobilized antibody. The 3 specific antigens, used as ligands in the marker combination, are respectively HCV antigen, HIV₁₊₂ antigen and syphilis antigen. The HCV Ag can specifically form a bi-antigen sandwich with the immobilized probe HCV Ag and the human HCV Ab captured by the probe HCV Ag. The HIV₁₊₂ Ag can specifically form a bi-antigen sandwich with the immobilized probe HIV₁₊₂ Ag and the human HIV₁₊₂ Ab captured by the probe HIV₁₊₂ Ag. The syphilis Ag can specifically form a bi-antigen sandwich with the immobilized probe syphilis Ag and the human syphilis Ab captured by the probe syphilis Ag. In this example, rhodamine is used as the label reagent and is bound to the ligands. The ligands are labeled with the rhodamine under optimized conditions, respectively. The markers prepared are ready for use in the form of a mixture or of two separated markers.

The negative control and the positive control of this chip kit are the same as those in comparative Example 1.

This biochip screening kit for blood transfusion can be used for detecting the possible presence of HBsAg, HCV antibody, HIV₁₊₂ antibody and syphilis antibody in a human serum sample. The human serum samples in this experiment are the same as those in comparative example 1. The detection of the samples in this example is performed in the same way as in comparative example 1, and the results obtained in the detection are similar to that in table 2 of comparative Example 1.

### Working Example 3:

### A biochip kit of detecting HIV antigen/antibody (1)

The kit prepared in this example consists of biochip probe-plate of detecting HIV antigen and HIV antibody, markers, negative controls, positive controls and washing buffer etc. The kit is to be used for detecting the possible presence of

HIV antigen and HIV antibody in a human serum sample.

The probe combination in this kit is that of one antibody and one antigen. The probe antibody selected is a mouse mAb against HIV p24 and the probe antigen selected is a mouse multiple-epitope-mixing antigen comprising HIV gp160, gp41, gp36. The probes at its optimized concentration are respectively spotted on a substrate in the form of a 2X3 micro-array (each probe spotted in 3 spots), and then bovine albumin is used to block residual surface of the substrate. The biochip probe-plate is then dried and ready for use.

The detection method combination used in the subject kit is that of bi-antigen sandwich detection and bi-antibody sandwich detection. The marker combination used in the subject kit is that of one labeled specific antibody and one labeled specific antigen. The specific antibody, used as ligand in the labeled specific antibody, is an anti-HIV p24 mAb. This anti-HIV p24 mAb is not directed against the probe HIV Ag epitope, but is directed against a sample HIV Ag captured by the probe HIV Ab in the form of a bi-antibody sandwich. The specific antigen, used as ligand in the labeled specific antigen, is a specific HIV Ag. This ligand HIV antigen does not react with the probe HIV Ab, but react with a sample HIV Ad captured by the probe HIV Ag in the form of a bi-antigen sandwich. In this example, rhodamine is used as the label reagent and is bound to the ligands. The ligands are labeled with the rhodamine under optimized conditions, respectively. The markers prepared are ready for use in the form of a mixture or of two separated markers.

The negative control in this chip kit is a HIV antibody and antigen negative human serum, selected by using ELISA kits. The ELISA probe-plates used in the ELISA kits are micro-well plates coated respectively with the anti-HIV p24 mAb and the mouse multiple-epitope-mixing antigen comprising HIV gp160, gp41, gp36. The positive control in this chip kit is a HIV antibody and antigen positive human serum, selected by using the ELISA kits.

This biochip kit can be used for testing the possible presence of HIV antibody and HIV antigen in a human serum sample. The human serum samples in this experiment are: A. HIV p24 antigen positive human serum, B. HIV gp160, gp41, gp36 antibody positive human serum, C.HIV antigen and antibody positive human serum, D. negative control, E. positive control. These human serum samples are previously detected by using the ELISA kits mentioned above.

The samples are detected as following: one of the human serum samples is diluted and subjected into one reactor of this chip. The antigen-antibody reaction in the reactor is performed at 37°C for one hour. Then, the unbound serum component is removed from the reactor and the reactor is washed with washing buffer. The marker combination is then subjected into the reactor. The marking reaction is performed at 37°C for one hour. Then, the reactor is washed first with buffer solution to get rid of unbound markers, and then with anhydrous alcohol. Finally, the dried biochip probe-plate is scanned and analyzed with a chip scanner (GMS 418 ARRAY SCANNER). The results of the detections with this chip kit are showed in the Table 3.

**Table 3: The results of HIV antigen/antibodies detecting biochip kit**

| Sample | results using 2 ELISA kits | | results using the biochip kit | |
|---|---|---|---|---|
| | Ab coated well | Ag coated well | Probe Ab spot | Probe Ag spot |
| A | +* | -* | + | - |
| B | - | + | - | + |
| C | + | + | + | + |
| Negative control | - | - | - | - |
| Positive control | ++++ | ++++ | ++++ | ++++ |
| Blank | - | - | - | - |

| | | | | |
|---|---|---|---|---|
| *: same as those in Table 1 | | | | |

### Comparative Example 4:

### A biochip kit of detecting HIV antigen/antibody (2)

The kit prepared in this comparative example consists of biochip probe-plate of detecting HIV antigen and HIV antibody, markers, negative controls, positive controls and washing buffer etc. The kit is to be used for detecting the possible presence of HIV antigen and HIV antibody in human serum sample.

The biochip probe-plate in this biochip kit is prepared by the same preparation method as that in comparative example 1.

The detection method used in the subject kit is a combination method of indirect detecting method and bi-antibody sandwich detection. The marker combination used in the subject kit is that of one labeled specific antibody and one labeled species-specific anti-antibody. The specific antibody, used as ligand in the marker combination, is a mouse mAb against HIV p24, which can specifically form a bi-antibody sandwich with the immobilized antibody against HIV P24 and human HIV Ag captured by the immobilized antibody. The anti-antibody, used as ligand in the anti-antibody, is a goat anti-antibody against human antibodies, which can bind with human HIV Ab captured by the immobilized HIV Ag, but cannot bind with the probe antibody (a mouse mAb against HIV p24 used as probe) and the labeled antibody (a mouse mAb against HBsAg used as marker ligand). In this example, rhodamine is used as the label reagent and is bound to the ligands. The ligands are labeled with the rhodamine under optimized conditions, respectively. The markers prepared are ready for use in the form of a mixture or of two separated markers.

The negative control and positive control of this chip kit are the same as those in example 3.

The human serum samples in this experiment are the same as those in example 3. The detection of the samples is performed in this example same as that in comparative example 1, and the results obtained in the detection are similar to that in table 3 of example 3.

### Comparative Example 5:

### A biochip kit for testing antigen/antibody related to tumors

The biochip kit prepared in this comparative example consists of biochip probe-plate, labeled material, negative control, positive control, reference materials, washing solution and etc. The biochip kit in this comparative example is to be used for detecting the possible presence of antigens and antibodies related to tumors in human serum samples.

The probe combination in biochip probe-plate in this biochip kit is determined depending on the detection requirement and the available tumor related antigens/antibodies. In this comparative example, the probe combination is antibody and antigen combination. The antigen probe in the probe combination is EBV antigen of synthetic polypeptide. The antibody probes in the probe combination are six mouse antibodies respectively against HCG trophic hormone, tumor marker antigen 50 (CA50), saccharide antigen 242 (CA242), mammary cancer specific antigens (CA153), carcinoembryonic antigen (CEA) and oophoroma specific antigen (CA125). The antigen and antibody probes at optimal concentration are respectively immobilized on the activated substrate in a form of 7 x 3 array (3 spots for each type of probe) by using a manual spotter. After spotting, bovine albumins is used to block the activated surface of the matrix. Then the biochip probe-plate is dried and ready for use.

The detection method combination used in the subject kit is that of indirect detecting method and bi-antibody sandwich detection. The marker combination used in the subject kit is that of labeled species-specific anti-antibody and labeled specific antibody. The first marker is used for the indirect detection method and the second marker, for the bi-antibody sandwich detection method. The specific antibodies in the marker combination are six mouse mAbs respectively against HCG, CA50, CA242, CA153, CEA and CA125, each of them can form a bi-antibodies sandwich with the immobilized antibody and its capturing antigen in the sample. The species-specific Anti-antibody in the marker combination is an anti-antibody against human IgA, which can bind to the EBV antibody captured by the immobilized EBV antigen. In this example, rhodamine is used as the label reagent and is paird on the ligands. The rhodamination of the ligands is realized respectively under optimized conditions. And then, the makers can be used in a mixed or separated form.

The negative control in this kit is the negative human serum selected through 7 ELISA kits, each of which includes a microwell probe-plate coated with one of the following probes: synthetic polypeptides EBV VCA antigen, antibody against HCG, antibody against tumor marker antigen 50 (CA50), the antibodies against polysaccharide and glucoprotein, such as saccharide antigen 242 (CA242), antibody against mammary cancer specific antigen (CA153), antibody against carcinoembryonic antigen (CEA) and antibody against oophoroma specific antigen (CA125). The quality controls in this kit are the tumor references selected through other quantitative test.

By using this biochip kit, one antibody and six antigens related to the tumors in one sample can be quantitatively detected in one reactor. So the kit has advantage in saving time and money.

### Comparative Example 6:

### A biochip kit of detecting HBV antibody/antigen

The biochip kit prepared in this comparative example consists of biochip probe-plate of detecting antibody and antigen related to hepatitis B virus (HBV), markers, negative control, positive control, washing solution and etc. This kit is for the detection of hepatitis B virus (HBV) antigens/antibodies in human serum sample.

The probe combination used in the subject kit, designed according to the detection objective and the available HBV related antigens/antibodies, includes two antibodies and 3 antigens. The two probe antibodies, selected for the probe combination, are an isolated mouse mAb against human HBsAg (hepatitis B surface antigen) and an isolated mouse mAb against human HBeAb (hepatitis B e antigen), respectively. The three probe antigens, selected for the probe combination, are an isolated HBsAg (hepatitis B surface antigen), an isolated HBcAg (hepatitis B core antigen) and a isolated HBeAg (hepatitis B e antigen). The probes at its optimized concentration are respectively spotted on a substrate in the form of a 5X3 micro-array (each probe spotted in 3 spots), and then bovine albumin is used to inactivate residual surface of the substrate. The biochip probe-plate is then dried and ready for use.

The kit prepared in this comparative example is to be used for the detection of hepatitis B surface antigen, hepatitis B e antigen, hepatitis B surface antibodies IgG, hepatitis B surface antibodies IgM, hepatitis B e antibodies IgG, hepatitis B e antibodies IgM, hepatitis B c antibodies IgG, hepatitis B c antibodies IgM in a human serum sample.

The detection method combination used in the subject kit is that of indirect detecting method and bi-antibody sandwich detection. The marker combination used in the subject kit is that comprising two labeled specific antibodies, one labeled species-specific anti-IgG and one labeled species-specific anti-IgM. The two specific antibodies, used as ligands in the marker combination, are an isolated mouse mAb against HBsAg and an isolated mouse mAb against HBeAb. The ligand HBsAb is not directed against the probe HBsAg epitope, but is directed against a sample HBsAg captured by the probe HBsAb in the form of a bi-antibody sandwich. The ligand HBeAb is not directed against the probe HBeAg epitope, but is directed against a sample HBeAg captured by the probe HBeAb in the form of a bi-antibody sandwich. The species-specific anti-IgG, used as ligand in the marker combination, is a goat antibody against human IgG. The species-specific anti-IgM, used as ligand in the marker combination, is a goat antibody against human IgM. These goat anti-antibodies react respectively to human IgG and IgM antibodies captured by the immobilized antigen probes, but not bind to any mouse antibody used as probe in the probe combination or used as ligand in the marker combination. In this comparative example, rhodamine (excitation wavelength λ ex=546nm and emission wavelength λ em=575nm) is used as the label reagent for labeling the two specific antibodies and the goat anti-human IgG. However, Cy5 (excitation wavelength X ex =649nm and emission wavelength λ em= 670nm) is used as the label reagent for labeling the goat anti-human IgM. The ligands are labeled with the rhodamine and Cy5 under optimized conditions, respectively.

The negative control in this kit is the negative human serum selected through ELISA method. The positive control in this example is the mixture of positive human serum selected through ELISA detection. The positive samples in this experiment respectively are: 1#. hepatitis B surface antigen positive sample, 2#. hepatitis B e antigen positive sample, 3#. hepatitis B surface antibodies IgG positive sample, 4#. hepatitis B surface antibodies IgM positive sample, 5#. hepatitis B e antibodies IgG positive sample, 6#. hepatitis B e antibodies IgM positive sample, 7#. hepatitis B c antibodies IgG positive sample, 8#. hepatitis B c antibodies IgM positive sample. The negative and positive results of all these selected human serum samples are pre-examined through ELISA.

The samples are detected as following: one of the human serum samples is diluted and subjected into one reactor of this chip. The antigen-antibody reaction in the reactor is performed at 37°C for one hour. Then, the unbound serum component is removed from the reactor and the reactor is washed with washing buffer. The marker combination (the mouse antibody against HBsAg labeled with rhodamine, the mouse antibody against HBeAg labeled with rhodamine, the goat anti-human IgG labeled with rhodamine, an the goat anti-human IgM labeled with Cy5) is then subjected into the reactor. The marking reaction is performed at 37°C for one hour. Then, the reactor is washed first with buffer solution to get rid of unbound markers, and then with anhydrous alcohol. Finally, the dried biochip probe-plate is scanned and analyzed with a multi-wavelength laser confocal microscopy (GMS 418 ARRAY SCANNER). The excitation wavelength of 540nm is used to obtain the detection results of hepatitis B surface antigen, hepatitis B e antigen, hepatitis B surface antibodies IgG, hepatitis B e antibodies IgG, hepatitis B c antibodies IgG. And the excitation wavelength of 650nm is used to detect hepatitis B surface antibody IgM, hepatitis B e antibody IgM, hepatitis B c antibody IgM. (Table 4):

**Table 4: The results of biochip kit for the detection of hepatitis B in human serum samples**

| Sample | Results using 8 ELISA kits | | | | | | | | Results using one biochip kit | | | | | | | |
|---|---|---|---|---|---|---|---|---|---|---|---|---|---|---|---|---|
| | A | B | C | D | E | F | G | H | A | B | C | D | E | F | G | H |
| 1# | + | -* | - | - | - | - | - | - | + | - | - | - | - | - | - | - |
| 2# | - | + | - | - | - | - | - | - | - | + | - | - | - | - | - | - |
| 3# | - | - | + | - | - | - | - | - | - | - | + | - | - | - | - | - |
| 4# | - | - | - | + | - | - | - | - | - | - | - | + | - | - | - | - |
| 5# | - | - | - | - | + | - | - | - | - | - | - | - | + | - | - | - |
| 6# | - | - | - | - | - | + | - | - | - | - | - | - | - | + | - | - |
| 7# | - | - | - | - | - | - | + | - | - | - | - | - | - | - | + | - |
| 8# | - | - | - | - | - | - | - | + | - | - | - | - | - | - | - | + |
| Negative control | - | - | - | - | - | - | - | - | - | - | - | - | - | - | - | - |
| Positive control | + | + | + | + | + | + | + | + | + | + | + | + | + | + | + | + |
| Blank | - | - | - | - | - | - | - | - | - | - | - | - | - | - | - | - |

| | | | | | | | | | | | | | | | | |
|---|---|---|---|---|---|---|---|---|---|---|---|---|---|---|---|---|
| * : same as those in Table 1 Notes: A:HbsAg; B:HBeAg; C:HbsIgG; D:HbsIgM; E:HbeIgG; F:HbeIgM; G:HbcIgG; H:HBcIgM | | | | | | | | | | | | | | | | |

### Comparative Example 7:

### A biochip kit of detecting HBV and HCV antigen/antibody

This kit is for detecting antigen/antibody of hepatitis B virus (HBV) and hepatitis C virus (HCV) in human serum sample.

In this comparative example, the kit consists of biochip probe-plate, markers, negative control, positive control, washing solution and etc.

Selection of the probe combination in the biochip probe-plate depends on the requirement of detection and the available related antigen/antibody. In this example, the antibody/antigen combination is selected as the probe combination.

The antibody probe in the probe combination is rabbit antibody against HBsAg, and the antigen probe in the probe combination is hepatitis C fusion antigen. The antibody probe is used for detecting hepatitis B surface antigen and the antigen probe for detecting hepatitis C antibody in a human serum sample. The antigen and antibody probes at optimal concentration are respectively immobilized on the activated substrate in a form of 2 x 3 array (3 spots for each type of probe) by using a manual spotter. Then, bovine albumin is used to block the surface of the matrix. The biochip probe-plate is then dried and ready for use.

Selection of the marker combination in this kit depends on the probe combination and also on the detection method to be used, which the last is selected as a combination of the bi-antibody sandwich detection method for antigen detection and the indirect detection method for antibody detection. So, the labeled anti-antibody with species-specificity and labeled specific antibody are selected as the marker combination. In the marker combination, the first marker is used for the indirect detection of the captured antibody and the second marker, for the bi-antibody sandwich detection of the captured antigen. The antibody ligand in the marker combination is a mouse mAb against HBsAg which can form a bi-antibody sandwich with the immobilized antibody probe ( mouse mAb against HBsAg) and its capturing HBsAg. The anti-antibody ligand with species-specificity is a goat Anti-antibody against human immunoglobulin, which can bind with the human HCV antibody captured by the antigen probe. The label reagent paird on the label ligands is rhodamine. The other accessory parts of the kit prepared in this comparative example are negative control, positive control and washing solution, etc.

### Comparative Example 8:

### A biochip kit of detecting hepatitis virus antigen/antibody

This kit prepared in the comparative Example consists of biochip probe-plate, markers, negative control, positive control, washing solution and etc. This kit is for detecting antibody and antigen related to different hepatitis virus.

The probe combination used in the subject kit, designed according to the detection objective and the available significative antigen and antibody related to different hepatitis virus, includes 1 antibody and 5 antigens. The probe antibody, selected for the probe combination, is an isolated rabbit mAb against human HBsAg (hepatitis B surface antigen). The five probe antigens, selected for the probe combination, are a HAV Ag (hepatitis A antigen), a multiple-epitope-fusion HCV Ag (hepatitis C virus antigen), a multiple-epitope-fusion HDV Ag (hepatitis D virus antigen), a multiple-epitope-fusion HGV Ag (hepatitis G virus antigen), and a multiple-epitope-fusion HEV Ag (hepatitis E virus antigen). The probes at its optimized concentration are respectively spotted on a substrate in the form of a 6X3 micro-array (each probe spotted in 3 spots), and then bovine albumin is used to inactivate residual surface of the substrate. The biochip probe-plate is then dried and ready for use.

The kit prepared in this comparative example is to be used for the detection of HAV antibody, HBs antigen, HCV antibody, HDV antibody and HEV antibody in a human serum sample.

The detection method combination used in the subject kit is that of indirect detecting method and bi-antibody sandwich detection. The marker combination used in the subject kit is that comprising one labeled specific antibody and one labeled species-specific anti-antibody. The specific antibody, used as ligand in the marker combination, is a rabbit mAb against HBsAg, which can specifically form a bi-antibody sandwich with the immobilized rabbit antibody against HBsAg and human HBsAg captured by the immobilized antibody. The anti-antibody, used as ligand in the anti-antibody, is a goat anti-antibody against human antibody, which can bind with human antibody, captured by their pair probe antigens (HAV Ag, HCV Ag, HDV Ag, HGV Ag and HEV Ag) immobilized respectively, but cannot bind with the rabbit probe antibody and the labeled rabbit antibody. In this example, rhodamine (excitation wavelength λ ex=546nm and emission wavelength λ em=575nm) is used as the label reagent and is bound to the ligands. The ligands are labeled with the rhodamine under optimized conditions, respectively. The markers prepared are ready for use in the form of a mixture or of two separated markers.

Other accessory parts of the kit prepared in this comparative example are negative control, positive control and washing solution, etc.

### Comparative Example 9:

### A rapid detection kit for blood transfusion screening

The kit fabricated in this comparative example is for the rapid detection of HBsAg, antibody against HCV, antibody against HIV₁₊₂, and antibody against syphilis present in a human serum sample. So it is applicable to rapid screening of blood donators.

This kit consists of a test strip, washing solution and etc. The test strip in this kit consists of a nitrocellulose membrane strip and a sample-loading part, a marker part, a probe part and a quality control part, which all of these parts are on the membrane strip. There is a glass fiber membrane in sample-loading area.

Selection of the probe combination in the probe-plate depends on the requirement of detection, for example the requirement according to the laws for blood transfusion in China. In this comparative example, antibody/antigen combination is selected as the probe combination, which is immobilized in the probe part. In the probe combination, the antibody probe is a rabbit antibody against HBsAg, and the antigen probes are HCV fusion antigen from mouse source, HIV₁₊₂ mixed antigen from mouse source and syphilis antigen from mouse source, respectively.

Each type of the probe is immobilized on the probe part in form of a detecting line.

The detection pattern to be used in the comparative example is selected as a combination of the bi-antibody sandwich detection pattern for antigen detection and the indirect detection pattern for antibody detection. So, the labeled anti-antibody with species-specificity and labeled specific antibody are selected as the marker combination. In the marker combination, the antibody ligand is rabbit antibody against HBsAg, and the Anti-antibody ligand is goat Anti-antibody against human immunoglobulin. The ligands are labeled respectively with colloidal gold, and then mixed, and finally immobilized on the membrane strip in form of line.

The maker part is located between the sample-loading part and the probe part. In this example, a quality control line of human IgG is immobilized in test strip area.

The human serum samples in this comparative example are the same as those in comparative example 1.

The rapid selection of the blood donators is different from the clinical diagnosis for patients. This kit can be used for the primary selection of blood donators within several minutes. When any positive result of a plasma sample is observed, the blood donor will be refused for the donation. Then the qualified donors will have further examinations. This way of selection can save time and reduce costs.

### Comparative Example 10:

### A rapid detection kit for HIV antigen/antibody

The kit fabricated in this comparative example can be used for the rapid detection of HIV antigen and antibody in human serum samples.

This kit consists of a test strip for rapid detection of the HIV antigen and antibody, washing solution and etc.

The test strip in this kit consists of a nitrocellulose membrane strip and a sample-loading part, a marker part, a probe part and a quality control part, which all of these parts are on the membrane strip. There is a glass fiber membrane in sample-loading area.

In this comparative example, the antibody/antigen combination is selected as the probe combination, which is immobilized in the probe part. In the probe combination, the antibody probe is mouse mAb against HIV p24, and the antigen probe is fusion protein of HIV gp160, gp41 and gp36 from mouse source, respectively. Each type of the probe is immobilized on the probe part in form of a detecting line.

The detection pattern to be used in the comparative example is selected as a combination of the bi-antibody sandwich detection pattern for antigen detection and the bi-antigen sandwiches detection pattern for antibody detection. So, the labeled specific antigen and labeled specific antibody are selected as the marker combination. In the marker combination, the antibody ligand is a mouse mAb against HIV p24, and the antigen ligand is a fusion antigen of HIV gp160, gp41 and gp36. The ligands are labeled respectively with colloidal gold, and then mixed, and finally immobilized on the membrane strip in form of line. The maker part is located between the sample-loading part and the probe part. In this example, a quality control line of Anti-antibodies against human immunoglobulin is immobilized in test strip area.

The human serum samples in this experiment are the same as those in example 3.

This rapid detection kit can save time and reducing detection cost since the detective results can be obtained and identified by eyes within several minutes.

### Comparative Example 11:

### A rapid detection kit for hepatitis

The kit fabricated in this comparative example can be used for the rapid identification of the hepatitis in a sample from a suspect hepatitis patient.

This kit consists of a test strip for the rapid detection, washing solution and etc. The test strip in this kit consists of a nitrocellulose membrane strip and a sample-loading part, a marker part, a probe part and a quality control part, which all of these parts are on the membrane strip. There is a glass fiber membrane in sample-loading area.

In this comparative example, the antibody/antigen combination is selected as the probe combination, which is immobilized in the probe part. In the probe combination, the antibody probe is a mouse antibody against human HBsAg, and the antigen probes are HAV antigen, HCV fusion antigen, HDV fusion antigen, a HEV fusion antigen and a HGV fusion antigen, respectively. Each type of the probe at a optimal concentration is immobilized on the probe part in form of a detecting line (forming 6 detective lines respectively). The kit also contains a quality control line of human IgG.

The detection pattern to be used in the example is selected as a combination of the bi-antibody sandwich detection pattern for antigen detection and the indirect detection pattern for antibody detection. So, the labeled anti-antibody with species-specificity and labeled specific antibody are selected as the marker combination. In the marker combination, the antibody ligand is a purified mouse mAb against HBsAg, and the species-specific anti-antibody ligand, a goat anti-human anti-Ab. The ligands are labeled with colloidal gold respectively, and then mixed, and finally immobilized on the membrane strip in form of line. The maker part is located above the probe part.

This kit can detect HAV antibody, hepatitis B surface antigen, HCV antibody, HDV antibody, HEV antibody and HGV antibody rapidly. The results can be identified by eyes. So the kit can save time and money.

### Comparative Example 12:

### A preparation of ELISA kit for blood transfusion rapid screening

In this comparative example, the kit consists of a micro-well plate coated with some antigens/antibodies concerned to the blood transfusion, markers and substrate, negative control, positive control, washing solution, etc.

The micro-well plate coated comprises a 96-wells-plate and the probe coated on the inner surface of the well. Selection of the probe combination in the probe-plate depends on the requirement of detection, par example the requirement according to laws for blood transfusion in China. In this example, the antibody/antigen combination is selected as the probe combination, which is immobilized in the probe part. In the probe combination, the antibody probe is a rabbit antibody against HBsAg, and the antigen probes are a HCV fusion antigen from mouse source, a HIV₁₊₂ mixed antigen from mouse source and a syphilis antigen from mouse source, respectively. These probes at optimal concentration are mixed at optimal proportion. Then the micro-wells are coated with the probe mixture on their inner surface, and then blockd with bovine albumin, and finally dried for use.

The labeling system in this chip kit is composed of makers and substrate (substrate coloration solution A and B). The detection pattern to be used in the example is selected as a combination of the bi-antibody sandwich detection pattern for antigen detection and the indirect detection pattern for antibody detection. So, the labeled anti-antibody with species-specificity and labeled specific antibody are selected as the marker combination. In the marker combination, the antibody ligand is a rabbit antibody against HBsAg, which can form the bi-antibody sandwiches with the immobilized rabbit antibody probe for capturing HbsAg, and the Anti-antibody ligand is a goat anti-antibody against human immunoglobulin, which can bind to human antibodies captured by the immobilized antigen probes, but not bind to the rabbit antibody probe against HBsAg and the labeled mouse mAb against HBsAg. The antibodies captured are human antibody against HCV, human antibody against HIV₁₊₂ and human antibody against syphilis, which are respectively captured by the HCV antigen probe, HIV₁₊₂ antigen probe and syphilis antigen probe. The ligands are labeled respectively with the enzyme, and then mixed at optimal concentration and optimal proportion.

The negative and positive control in this kit in the comparative example are the same as those in example 1.

This kit can be used to detect whether a human serum example is negative for all of the target molecules, or is positive for anyone of the target molecules. Said target molecules are HBsAg, HCV antibody, HIV₁₊₂ antibody and syphilis antibody, respectively. The rapid selection of the blood donators is different from the clinical diagnosis for patients. This kit can be used for the primary selection of blood donators within several minutes. When any positive result of a plasma sample is observed, the blood donor will be refused for the donation.

Although this chip kit cannot be applied to identifying one of the target molecules, it is still advantageous to clinic, since it can screen blood donors rapidly and economically.

In this comparative example, the following samples are used for the detection by this kit: 3 negative controls, 3 positive controls, 1 blank, and 8 samples selected. After diluted, these samples except the blank are respectively added in 14 wells and react with the probe combination coated on the well. After the reaction, the labeled anti-antibody with species-specificity and labeled specific antibody are added in all of the wells, and then the substrate solution and the reaction stop solution are also added. Then results are read with a ELISA reader and showed in the Table 5:

**Table 5: the results of rapid selection of blood donators**

| Samples | Results using the present ELISA kit* | | | | Results of the Kit in the example ** |
|---|---|---|---|---|---|
| | HBsAg | HCV | HIV | Syphilis | |
| 1 | + | - | - | - | + |
| 2 | - | + | - | - | + |
| 3 | - | - | + | - | + |
| 4 | - | - | | + | + |
| 5 | - | - | | | + |
| 6 | + | + | - | - | ++ |
| 7 | - | - | + | + | ++ |
| 8 | + | - | - | - | ++ |
| Negative control | - | - | - | - | - |
| Positive control | + | + | + | + | +++ |
| Blank | - | - | - | - | - |

## Claims

1. A biochip kit, comprising:
• one biochip with at least one reactor, in which at least one probe antibody and one probe antigen are immobilized non-randomly in the reactor in the form of a probe micro-array, wherein said probe antibody includes a mouse antibody against anti-HIV p24 antigen and said probe antigen is a mouse multiple-epitope-mixing antigen comprising HIV gp36, gp41 and gp160; and
• marker, wherein said marker consists of ligands, corresponding to said probe antibody and probe antigen, and label reagent bound to the ligands.

2. The kit of claim 1, wherein said marker is one of the following marker combinations:
A. the combination of labelled specific antibody and labelled specific antigen;
B. the combination of labelled anti-antibody with species-specificity and labelled specific antibody and/or labelled specific antigen;
C. the combination of labelled anti-antibody with structure-specificity and labelled specific antibody and/or labelled specific antigen;
D. any combination derived from the combinations mentioned above.

3. A method for biological sample analysis with a biochip, comprising:
(1) subjecting a biological sample to the reactor in the biochip kit of either of claims 1 or 2, and making it react;
(2) optionally, subjecting marking reagents to said reactor in the form of an individual substance, a partial mixture or a complete mixture, and
(3) analyzing results in the reactor after the reaction.

## Patentansprüche

1. Biochip-Kit, umfassend
• einen Biochip mit mindestens einem Reaktor, wobei mindestens ein Sonden-Antikörper und ein Sonden-Antigen auf nicht zufällige Weise im Reaktor in Form eines Sonden-Microarrays immobilisiert sind, wobei der Sonden-Antikörper einen Maus-Antikörper gegen das anti-HIV p24-Antigen einschließt und es sich bei dem Sonden-Antigen um ein Maus-Mischantigen mit multiplen Epitopen handelt, das gp36, gp41 und gp160 von HIV umfasst; und
• Marker, wobei der Marker aus Liganden besteht, die dem Sonden-Antikörper und Sonden-Antigen entsprechen, und Markierungsreagens, das an die Liganden gebunden ist.

2. Kit nach Anspruch 1, wobei es sich bei dem Marker um eine der folgenden Markerkombinationen handelt:
(A) Kombination von markiertem, spezifischem Antikörper und markiertem, spezifischem Antigen;
(B) Kombination von markiertem anti-Antikörper mit Spezies-Spezifität und markiertem, spezifischem Antikörper und/oder markiertem, spezifischem Antigen;
(C) Kombination von markiertem anti-Antikörper mit Strukturspezifität und markiertem, spezifischem Antikörper und/oder markiertem, spezifischem Antigen;
(D) jede aus den vorstehend erwähnten Kombinationen abgeleitete Kombination.

3. Verfahren zur biologischen Probenanalyse mit einem Biochip, umfassend:
(1) eine biologische Probe in dem Biochip-Kit nach einem der Ansprüche 1 oder 2 dem Reaktor auszusetzen und sie reagieren zu lassen;
(2) gegebenenfalls Markierungsreagenzien in Form einer einzelnen Substanz, eines teilweisen Gemisches oder eines vollständigen Gemisches dem Reaktor auszusetzen und
(3) Ergebnisse nach der Reaktion in dem Reaktor zu analysieren.

## Revendications

1. Kit de biopuce comprenant :
• une biopuce avec au moins un réacteur, dans laquelle au moins un anticorps de sonde et un antigène de sonde sont immobilisés de façon non aléatoire dans le réacteur sous la forme d'un microréseau de sondes, dans laquelle ledit anticorps de sonde contient un anticorps de souris dirigé contre l'antigène p24 anti-VIH et ledit antigène de sonde est un antigène de mélange d'épitopes multiples de souris comprenant gp36, gp41 et gp160 de VIH ; et
• un marqueur, ledit marqueur étant constitué de ligands, correspondant audit anticorps de sonde et audit antigène de sonde, et un réactif marqueur lié aux ligands.

2. Kit selon la revendication 1, dans lequel ledit marqueur est l'une des combinaisons de marqueurs suivantes :
A. la combinaison d'anticorps spécifique marqué et d'antigène spécifique marqué ;
B. la combinaison d'anti-anticorps marqué ayant une spécificité d'espèce et d'anticorps spécifique marqué et/ou d'antigène spécifique marqué ;
C. la combinaison d'anti-anticorps marqué ayant une spécificité de structure et d'anticorps spécifique marqué et/ou d'antigène spécifique marqué ;
D. n'importe quelle combinaison issue des combinaisons mentionnées ci-dessus.

3. Procédé d'analyse d'un échantillon biologique au moyen d'une biopuce comprenant :
(1) l'opération consistant à soumettre un échantillon biologique au réacteur dans le kit de biopuce de l'une ou l'autre des revendications 1 et 2, et à le faire réagir ;
(2) éventuellement, l'opération consistant à soumettre des réactifs de marquage audit réacteur sous la forme d'une substance individuelle, d'un mélange partiel ou d'un mélange complet, et
(3) l'analyse des résultats dans le réacteur après la réaction.
